# EUROPEAN PATENT APPLICATION

(11) **EP 1 321 159 A1**
(43) Date of publication of application: **25.06.2003**
(21) Application number: 01130521.6
(22) Date of filing: 21.12.2001
(51) Int. Cl.: A61M 15/00, A61K 9/00

(54) **Pressurized metered dose inhaler (pMDI) actuators with laser drilled orifices**

(71) Applicant: CHIESI FARMACEUTICI S.p.A., I-43100 Parma (IT)
(72) Inventor: Davies, Rebecca Jayne, Parma (IT); Ganderton, David, Parma (IT); Lewis, David Andrew, Parma (IT); Meakin, Brian John, Parma (IT)
(74) Representative: Adam, Holger, Dr.

(57) **Abstract**

The present invention relates to pressurized metered dose inhaler (pMDI) actuators with laser drilled orifices and to medicinal aerosol solution formulation products comprising these actuators. In particular, the present invention relates to the optimisation of the output characteristics of drug solution formulations in hydrofluoroalkanes (HFAs) by use of pMDIs with actuators with laser drilled orifices. Moreover, the actuators of the present invention allow the use of solution formulations with a high ethanol content and thus, the use of poorly soluble active ingredients in solution formulations.

## Description

The present invention relates to pressurized metered dose inhaler (pMDI) actuators with laser drilled orifices and to medicinal aerosol solution formulation products comprising these actuators. In particular, the present invention relates to the optimisation of the output characteristics of drug solution formulations in hydrofluoroalkanes (HFAs) by use of pMDIs with actuators with laser drilled orifices. Moreover, the actuators of the present invention allow the use of solution formulations with a high ethanol content and thus, the use of poorly soluble active ingredients in solution formulations.

The pharmaceutical solution formulations in hydrofluoroalkanes used in the present invention may be filled into canisters suitable for delivering pharmaceutical aerosol formulations. Canisters generally comprise a container capable of withstanding the vapour pressure of the HFA propellant, such as plastic or plastic-coated glass bottle or preferably a metal can, for example a stainless steel can or aluminium can which is preferably anodised, organic coated, such as lacquer-coated and/or plastic coated (WO00/30608 of the applicant), which container is closed with a metering valve. The metering valves are designed to deliver a metered amount of the formulation per actuation and incorporate a gasket to prevent leakage of propellant through the valve. The gasket may comprise any suitable elastomeric material such as for example low density polyethylene, chlorobutyl, black and white butadiene-acrylonitrile rubbers, butyl rubber and neoprene. Thermoplastic elastomer valves as described in WO92/11190 and valves containing EPDM rubber are especially suitable. Suitable valves are commercially available from manufacturers well known in the aerosol industry, for example, from Valois, France (e.g. DF10, DF30, DF31, DF60), Bespak plc UK (e.g. BK300, BK356, BK357) and 3M-Neotechnic Ltd. UK (e.g. Spraymiser™).

Valve seals, especially the gasket seal, and also the seals around the metering chamber, will preferably be manufactured from a material which is inert to and resists extraction into the contents of the formulation, especially when the contents include ethanol.

Valve materials, especially the material of manufacture of the metering chamber, will preferably be manufactured of a material which is inert to and resists distortion by contents of the formulation, especially when the contents include ethanol. Particularly suitable materials for use in manufacture of the metering chamber include polyesters eg polybutyleneterephthalate (PBT) and acetals, especially PBT.

Materials of manufacture of the metering chamber and/or the valve stem may desirably be fluorinated, partially fluorinated or impregnated with fluorine containing substances in order to resist drug deposition.

Each filled canister is conveniently fitted into a suitable channelling device prior to use to form a metered dose inhaler for administration of the medicament into the lungs or nasal cavity of a patient. Suitable channelling devices comprise, for example a valve actuator and cylindrical or conelike passage through which medicament may be delivered from the filled canister via the metering valve to the nose or mouth of a patient e.g. a mouthpiece actuator.

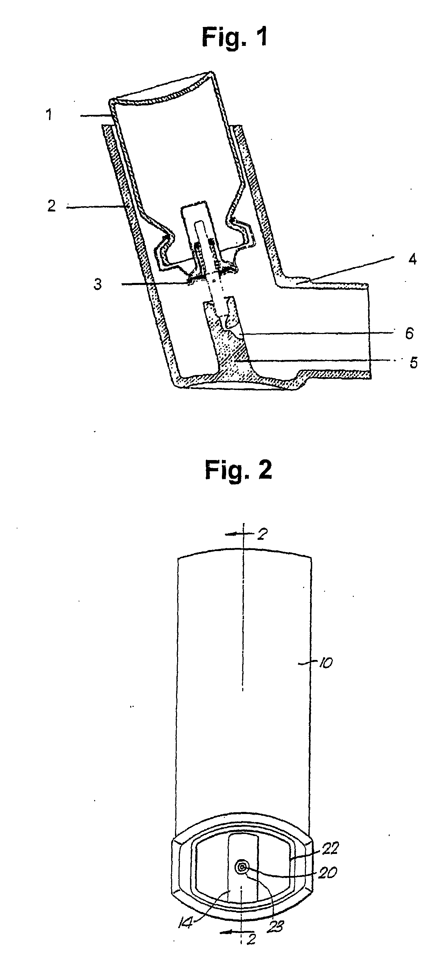

In a typical arrangement (Figure 1) the valve stem is seated in a nozzle block which comprises an actuator insert 5, which comprises an actuator orifice 6 leading to an expansion chamber. Conventional pressurized metered dose inhaler actuators have variable actuator orifice diameters from 0.25 to 0.42mm and a length of 26mm (Bespak typ BK632). In other types of actuators the lengths can vary. International Patent Application WO01/19342 discloses actuator orifice diameters in the range of 0.15 to 0.45mm, particularly 0.2 to 0.45mm. According to this prior art reference it is advantageous to use a small diameter e.g. 0.25mm or less, particularly 0.22mm since this tends to result in a higher FPM (fine particle mass) and lower throat deposition. Moreover it is stated that 0.15mm is also particularly suitable. However, this prior art reference does not disclose how to obtain actuator orifices of less than 0.2mm. The examples only relate to pMDIs having actuator orifices of 0.22mm, 0.33mm and 0.50mm. Thus, although referring in general to small actuator orifice diameters of less than 0.2mm, the prior art does not provide a solution how to obtain such small orifices with a high precision, i.e. with tightly controlled tolerances.

Actuator orifice lengths are typically in the range from 0.30 to 1.7mm, e.g. 0.30, 0.65 or 1.50mm.

Metered dose inhalers are designed to deliver a fixed unit dosage of medicament per actuation or "puff", for example in the range of 25 to 250µg medicament per puff.

In the accompanying drawings, figure 1 shows a conventional pressurized metered dose inhaler comprising a canister 1, an actuator 2, a metering valve 3, an oral tube 4, and a nozzle block comprising an actuator insert 5 and an actuator orifice 6.

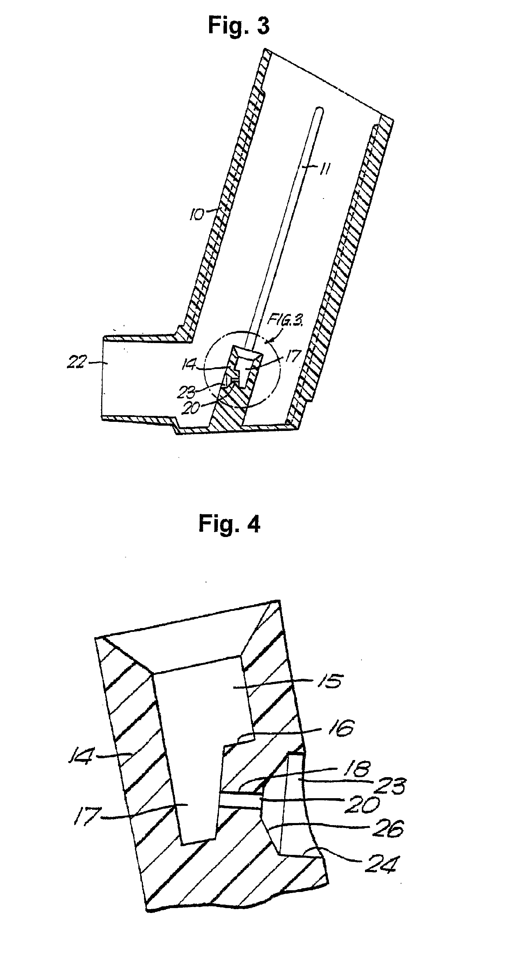

Figures 2, 3 and 4 show a conventional actuator nozzle block.
Figure 3 is a section on line 2-2 of figure 2, and figure 4 is an enlarged reversed view of the circled part of figure 3.

Referring to the figures, a conventional pressurized metered dose inhaler consists of a body portion 10 of an actuator into which a pressurized canister 1 containing a medicinal aerosol solution formulation may be inserted, and located by means of ribs 11.

A nozzle block 14 of the body portion 10 has a bore 15 which receives a valve outlet pipe of the canister 1. The end of the outlet pipe beares on a step 16 within the base so that compressing the body portion 10 and canister 1 together opens the valve 3 and causes the discharge under pressure of a single measured quantity of the drug in its carrier medium.

The dose passes down a passage 17 in the nozzle block 14, through a conduit 18 (with an actuator orifice length), e.g. a parallel-bore conduit to a discharge nozzle 20 (with an actuator orifice diameter), and thence through a mouthpiece 22 of the body portion 10 of the actuator.

The shape and direction of the discharge plume and the dispersion of the droplets or particles therein are critical to effective administration of a controlled dose to the patient.

Conventionally the discharge nozzle 20 is positioned in a cylindrical recess 23 in the nozzle block 14 having a parallel sided portion 24 and a frusto-conical base 26. In order for the patient to insert the mouthpiece at the correct orientation for discharge of the spray whilst at the same time holding the body portion 10 of the actuator and the canister 1 at a convenient angle, the axis of the mouthpiece 22 is inclined at an obtuse angle of about 105 degrees to that of the body portion 10 of the actuator and nozzle block 14. Because of this geometry, the conical recess is not perpendicular to the surface of the nozzle block 14, resulting in the parallel sided portion 24 being shorter on one side than the other.

The dimension of the discharge nozzle 20 (actuator orifice diameter) and the recess 23 are such that the discharge plume dose not impinge directly upon the sides of the recess 23.

A problem with known inhaler spray nozzles is that of adequately matching the dimensions of the conduit 18 (actuator orifice length) and nozzle 20 (actuator orifice diameter) to the particular drug formulation and carrier-propellant. Different drugs have different flow and dispersion characteristics (particularly as between powder and liquid formulations) and it is often difficult to achieve the optimum balance between the plume shape, total dose volume and discharge duration.

It has been disclosed (Lewis D.A. et al., Respiratory Drug Delivery VI, 363-364, 1998) that using commercially available actuators for delivering solution formulations of aerosol pressurized with HFA, the reduction in the orifice diameter induces an increase in the fine particle dose (FPD) of the aerosol produced.

FPD, which provides a direct measurement of the aerosol particles considered suitable for deposition and retention in the respiratory tract, is calculated as the mass of the particles deposited from stage 3 to filter (particles with an aerodynamic diameter less than 4.7µm) of the Andersen Cascade Impactor.

HFA solution formulations usually contain a co-solvent, generally an alcohol and specifically ethanol, to dissolve the active ingredient in the propellant. Depending on the concentration and solubility characteristics of the active ingredient, the concentration of solubilisation agent (e.g. ethanol) can increase. Large amounts of ethanol increase, proportionally to their concentration, the velocity of the aerosol droplets leaving the actuator orifice. The high velocity droplets extensively deposit into the oropharyngeal tract to the detriment of the dose which penetrates in the lower airways (i.e. respirable fraction or fine particle fraction (FPF)).

The technical problem underlying the present invention is to provide pressurized metered dose inhaler actuators with an optimisation of the output characteristics of drug solution formulations in hydrofluoroalkanes (HFAs). In particular, it is a technical problem underlying the present invention to provide actuators with an extremely efficient atomisation also with HFA solution formulations containing high levels of ethanol (at least 15% and up to 20 or 25% by weight or more) i.e. showing a fine particle fraction (i.e. particles with a diameter smaller than 4.7µm) of at least 50%.

These technical problems have been solved by an actuator as claimed in claim 1 and a medicinal aerosol solution formulation product according to claim 9.

According to the present invention an actuator is provided with an actuator orifice having a diameter in the range from 0.10 to 0.30mm, preferably 0.11 to 0.22mm and in particular from 0.11 to 0.14mm, wherein a diameter of 0.14mm is particularly preferred. The small actuator orifice diameters are obtained by using a laser to drill the actuator orifices. The advantages of using a laser to drill the actuator orifices include, very high precision down to a few microns, smooth interior bore, tightly controlled taper and dimensional tolerances, entry angle holes down to 10 degrees and minimal heat damage. Thus, the present invention provides for the first time an alternative to existing moulding technics and provides pMDI actuators with very small actuator orifice diameters with tightly controlled tolerances which is necessary to be able to provide tightly controlled reproduceability of the unit dosage of medicament per actuation.

For example a copper vapour laser (CVL) (Oxford Lasers ltd.) can be used to produce actuators with tightly controlled tolerances on orifice diameter and length.

In one embodiment of the invention the nozzle structure is manufactured as a separate actuator insert piece which is fitted into the nozzle block 14. Alternatively or in addition, the nozzle block may be a separate component fitted into the body portion 10.

Preferably, the actuator insert pieces are constructed of aluminium, as using a CVL to micro drill plastic results in to much heat damage. However, according to one embodiment of the invention it is possible to laser drill into plastics without heat damage, by frequency doubling the visible output of the CVL. This generates three ultra-violet wave lengths, e.g. 255nm, 271nm and 289nm. With these ultra-violet wave lengths plastics can be drilled to high precision without heat damage.

Any kind of actuator inserts known in the art, or of nozzle structures known in the art (e.g. as described in GB-A-2276101 and WO99/12596) can be provided with laser drilled orifices. Preferably, the actuator inserts or nozzle structures are made of aluminium.

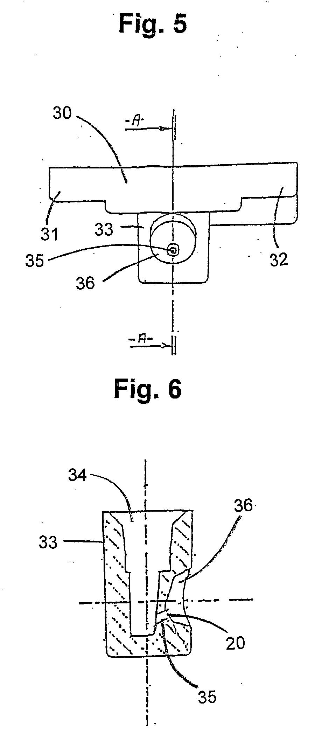

In one embodiment of the present invention an aluminium nozzle block known in the art as the "Chiesi Jet piece" is provided with a laser drilled orifice. Figures 5 and 6 show the dimensions of the "Chiesi Jet piece" used in the examples of the present invention. Figure 5 is a front view of the T shaped nozzle block. Figure 6 is a section view of the nozzle block along lines A-A of figure 5. The "Chiesi Jet piece" is a separate component fitted into the body portion 10. For a detailed description reference is made to international patent application WO99/12596.

The nozzle block (30) is shaped as a T, consisting of an upper bar composed by two fins (31, 32) to be housed and retained in two seats provided in the two shells forming the device and of a vertical stem (33) shorter than the horizontal upper bar.

The vertical stem (33) comprises a socket (34) provided with a seat to house a hollow stem of a pressurized can.

In the thickness of the stem (33) is bored a conduit (35) that connects the socket (34) with the expansion chamber of the device through the orifice (20) positioned in a recess (36).

### Examples

### Example 1

The "Chiesi Jet piece" was used as a model for the aluminium nozzle block in the examples of the present invention. Once drilled the aluminium nozzle block was housed in a modified Bespak 630 series actuator. Test pieces were also constructed and used to check the orifice entrance and exit diameters. Adjusting the laser power and focus controls the converging and diverging of the orifice. The dimensions of all actuator orifices were checked using a Mitntoyo TM WF20X microscope and Dolan-Jenner Fiberlite.

Table 1 shows the dimensions of a range of actuator orifice diameters from 0.10mm to 0.30mm, with 0.30mm orifice length (n=1) and 0.60 mm orifice length (n=2). The various shaped orifices that were produced are slot, cross, clover leaf and peanut, the dimensions of which are shown in table 2. Multiple holed actuator orifices were also produced. The dimensions of the multiple holed orifices are included in table 2.

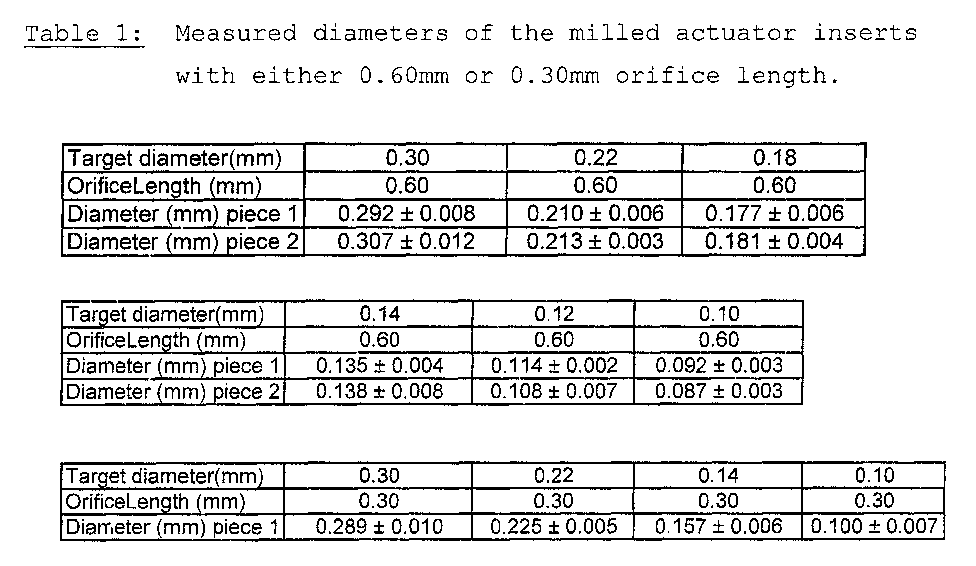

**Table 2:**

| Measured diameters of milled actuator inserts with either shaped or multiple holed orifices (* holes are 0.5mm apart). | | | | | | |
|---|---|---|---|---|---|---|
| Area Cf. (mm) | 0.33 | | 0.22 | | 0.22 | |
| Orifice Shape | Slot | | Cross | | clover leaf | |
| x (mm) | 0.042 ± 0.005 | 0.042 ± 0.003 | 0.531 ± 0.004 | 0.573 ± 0.004 | 0.244 ± 0.003 | 0.250 ± 0.002 |
| y (mm) | 2,051 | 1,984 | 0.539 ± 0.005 | 0.542 ± 0.002 | 0.242 ± 0.002 | 0.229 ± 0.003 |
| z (mm) | | | 0.036 ± 0.003 | 0.039 ± 0.003 | | |
| Area Cf. (mm) | 0.10 | | 0.22 | | 0.12 | 0.12 |
| Orifice Shape | Peanut | | peanut | | 2 hole* | 4 hole* |
| x (mm) | 0.068 ±0.003 | | 0.153 ±0.004 | 0.175 ±0.003 | 0.083 ±0.006 | 0.061 ±0.005 |
| y (mm) | 0.116 ±0.001 | | 0.276 ±0.005 | 0.295 ±0.004 | | |

| | | | | | | |
|---|---|---|---|---|---|---|
| x indicates the extension in the horizontal direction; | | | | | | |
| y indicates the extension in the vertical direction; | | | | | | |

Table 1 clearly shows the high precision that can be achieved with laser drilling into aluminium, as the measured orifice diameters closely match the target diameters. In table 2 the differences in the measurements between the two peanut shaped orifices with an area comparable to a 0.22mm orifice, can be explained by the number of laser drillings used to produce each of these peanut orifices. The smaller of the two was produced with one laser drilling, the larger was achieved with two drillings. All other shaped orifices, including the peanut with an area comparable to a 0.10mm conventional actuator were produced with two laser drillings.

### Example 2

The experiments of example 2 consisted of discharging beclometasone dipropionate (BDP)/ethanol/HFA 134a formulations , with and without glycerol, through the actuator insert housed in the modified Bespak actuator (630 series) into an Andersen Cascade Impactor operated at 28.3 Lmin⁻¹. Two product strengths, 50µg/dose (with 7% ethanol) and 250µg/dose (with 15% ethanol and 1.3% glycerol) were used. The drug deposited on the actuator, the throat and the stages of the impactor were measured. The delivered dose, the mass median aerodynamic diameter (MMAD), the geometric standard deviation (GSD), the fine particle dose ≤4.7µm (FPD_{≤4.7}) and the fine particle dose ≤1.1µm (FPD_{≤1.1}) were calculated. The FPDs were also expressed as a % fraction of the ex-valve dose (FPF_{≤4.7}, FPF_{≤1.1}). Shot weight was measured by weighing the pMDI before and after discharge.

The data for the clouds generated by a range of laser drilled orifice diameters all with 0.60mm length for the 250µg and 50µg BDP formula are given in Table 3a and 3b respectively. Table 4a and 4b gives the data generated for the 250µg and 50µg BDP formula with the laser drilled orifices with 0.30mm orifice length.

**Table 3a:**

| Data generated with BDP 250µg formula and a range of laser drilled orifice diameters all with 0.60mm length. | | | | | |
|---|---|---|---|---|---|
| Orifice Diameter (mm) | 0.30 | 0.22 | 0.18 | 0.14 | 0.12 |
| Length (mm) | 0.6 | 0.6 | 0.6 | 0.6 | 0.6 |
| Recovered (µg) | 252.22 | 250.11 | 246.84 | 250.96 | 251.38 |
| Delivered (µg) | 235.60 | 232.15 | 233.65 | 237.10 | 234.65 |
| Actuator (µg) | 16.63 | 17.94 | 13.17 | 13.88 | 16.74 |
| Throat (µg) | 135.47 | 92.06 | 53.38 | 23.65 | 31.24 |
| Stage 0 - 2 (µg) | 20.46 | 25.52 | 21.20 | 20.97 | 32.04 |
| Stage 0 - 2 (%) | 8.11 | 10.2 | 8.59 | 8.36 | 12.75 |
| FPD < 4.7µm (µg) | 79.67 | 114.59 | 159.1 | 192.47 | 171.37 |
| FPF<47 (%) | 31.59 | 45.82 | 64.45 | 76.69 | 68.17 |
| Dose < 1.1µm (µg) | 11.93 | 12.62 | 18.44 | 23.27 | 19.79 |
| FPF<1.1µm (%) | 4.73 | 5.04 | 7.47 | 9.27 | 7.87 |
| MMAD (µm) | 2.7 | 2.7 | 2.5 | 2.4 | 2.6 |
| GSD | 2.1 | 1.9 | 1.8 | 1.8 | 1.9 |
| Average Shot Weight | 57.8 ±0.7 | 58.5 ±0.8 | 57.1 ±0.7 | 58.1 ±0.5 | 55.4 ± 2.0 |

**Table 3b:**

| Data generated with BDP 50µg formula and a range of laser drilled orifice diameters all with 0.60mm length. | | | | | |
|---|---|---|---|---|---|
| Orifice Diameter (mm) | 0.30 | 0.22 | 0.18 | 0.14 | 0.12 |
| Length (mm) | 0.6 | 0.6 | 0.6 | 0.6 | 0.6 |
| Recovered (µg) | 50.24 | 51.97 | 49.97 | 50.35 | 49.77 |
| Delivered (µg) | 47.05 | 49.00 | 46.65 | 47.00 | 47.10 |
| Actuator (µg) | 3.23 | 2.95 | 3.33 | 3.39 | 2.66 |
| Throat (µg) | 15.49 | 9.07 | 4.45 | 3.50 | 3.01 |
| Stage 0 - 2 (µg) | 1.33 | 1.62 | 1.11 | 1.22 | 1.47 |
| Stage 0 - 2 (%) | 2.65 | 3.12 | 2.22 | 2.42 | 2.95 |
| FPD <4.7µm (µg) | 30.20 | 38.34 | 41.08 | 42.25 | 42.63 |
| FPF<47 (%) | 60.11 | 73.77 | 82.21 | 83.91 | 85.65 |
| Dose < 1.1 µm (µg) | 14.91 | 19.21 | 24.14 | 27.44 | 24.72 |
| FPF<1.1µm (%) | 29.68 | 36.96 | 48.31 | 54.50 | 49.67 |
| MMAD (µm) | 1.1 | 1.1 | 1.0 | 0.9 | 1.0 |
| GSD | 2.0 | 1.9 | 1.9 | 1.9 | 1.9 |
| Average Shot Weight | 59.8 ± 0.7 | 59.6 ± 0.7 | 61.7 ±0.5 | 60.2 ±0.5 | 59.4 ± 0.9 |

**Table 4a:**

| Data generated with BDP 250µg formula and a range of laser drilled orifice diameters all with 0.30mm length. | | | |
|---|---|---|---|
| Orifice Diameter (mm) | 0.30 | 0.22 | 0.14 |
| Length (mm) | 0.3 | 0.3 | 0.3 |
| Recovered (µg) | 265.66 | 261.45 | 254.91 |
| Delivered (µg) | 243.05 | 242.80 | 242.65 |
| Actuator (µg) | 22.63 | 18.64 | 12.27 |
| Throat (µg) | 136.75 | 100.16 | 27.58 |
| Stage O - 2 (µg) | 23.65 | 31.34 | 22.18 |
| Stage 0 - 2 (%) | 8.90 | 11.99 | 8.70 |
| FPD < 4.7µm (µg) | 82.64 | 111.32 | 192.89 |
| FPF<4.7 (%) | 31.11 | 42.58 | 75.67 |
| Dose < 1.1 µm (µg) | 12.39 | 12.97 | 22.23 |
| FPF<1.1 µm (%) | 8.66 | 4.96 | 8.72 |
| MMAD (µm) | 2.8 | 3.0 | 2.5 |
| GSD | 2.2 | 2.1 | 1.8 |
| Average Shot Weight | 58.0 ± 1.0 | 58.8 ± 0.5 | 57.7 ± 0.3 |

**Table 4b:**

| Data generated with BDP 50µg formula and a range of laser drilled orifice diameters all with 0.30mm length. | | | | | |
|---|---|---|---|---|---|
| | Orifice Diameter (mm) | 0.30 | 0.22 | 0.14 | |
| | Length (mm) | 0.3 | 0.3 | 0.3 | |
| | Recovered (µg) | 53.92 | 54.59 | 48.76 | |
| | Delivered (µg) | 48.70 | 50.45 | 45.25 | |
| | Actuator (µg) | 5.20 | 4.11 | 3.49 | |
| | Throat (µg) | 17.86 | 14.22 | 7.81 | |
| | Stage 0 - 2 (µg) | 2.52 | 3.21 | 4.43 | |
| | Stage 0 - 2 (%) | 4.67 | 5.88 | 9.09 | |
| | FPD < 4.7µm (µg) | 28.34 | 33.06 | 33.03 | |
| | FPF<4.7 (%) | 52.56 | 60.56 | 67.74 | |
| | Dose < 1.1µm (µg) | 14.90 | 15.17 | 21.35 | |
| | FPF<1.1µm (%) | 27.63 | 27.79 | 43.79 | |
| | MMAD (µm) | 1.2 | 1.4 | 1.2 | |
| | GSD | 2.4 | 2.3 | 2.9 | |
| | Average Shot Weight | 58.3 ±0.8 | 60.1 ±0.8 | 59.1 ± 5.05 | |

The data generated with the 0.60mm and 0.30mm orifice length actuators for the BDP 250µg and 50µg formula show a very clear increase in FPF_{≤4.7} as orifice diameter decreases . An optimum orifice diameter of 0.14mm is seen with the BDP 250µg formulation giving 76.69% FPF_{≤4.7}. The increase in FPF_{≤4.7} is accompanied by a decrease in throat deposition and MMAD as orifice diameter decreases. There is very little change in actuator deposition. No data was generated for the 0.10mm diameter orifice, as the orifice is too small for atomisation to occur.

Comparisons between the 0.60mm and 0.30mm orifice length show no differences for the BDP 250µg formula. However with the BDP 50µg formula greater FPF_{≤4.7} are achieved with the longer orifice length. For the 0.14mm diameter orifice a FPF_{≤4.7} of 83.91% is achieved with the 0.60mm length while a FPF_{≤4.7} of 67.74% is achieved with the 0.30mm orifice length.

### Example 3

The data generated for the different orifice shapes with the BDP 250µg and 50µg formula are shown in table 5a and 5b respectively. The data for the multiple orifice actuator inserts is shown in table 6.

**Table 5a:**

| Data generated with BDP 250µg formula and a range of shaped actuator orifices. | | | | | |
|---|---|---|---|---|---|
| Shape | Slot | Cross | Peanut | Peanut | Clover Leaf |
| Area comparable to (mm) | 0.33 | 0.22 | 0.22 | 0.1 | 0.22 |
| Recovered (µg) | 252.71 | 248.75 | 248.21 | 80.14 | 233.32 |
| Delivered (µg) | 222.90 | 233.90 | 236.30 | 11.95 | 219.15 |
| Actuator (µg) | 29.82 | 14.86 | 11.93 | 68.22 | 14.17 |
| Throat (µg) | 52.78 | 45.87 | 44.99 | 3.65 | 66.69 |
| Stage 0 - 2 (µg) | 38.57 | 31.48 | 31.70 | 1.58 | 21.22 |
| Stage 0 - 2 (%) | 15.26 | 12.66 | 12.77 | 1.97 | 9.09 |
| FPD < 4.7µm (µg) | 131.56 | 156.55 | 159.60 | 6.70 | 131.25 |
| FPF<4.7 (%) | 52.06 | 62.93 | 64.30 | 8.36 | 56.25 |
| Dose<1.1µm(µg) | 17.02 | 19.06 | 14.21 | 2.00 | 13.78 |
| FPF<1.1µm (%) | 6.73 | 7.66 | 5.72 | 2.50 | 5.91 |
| MMAD (µm) | 2.8 | 2.6 | 2.7 | (2.1) | 2.6 |
| GSD | 2.0 | 1.9 | 1.8 | (2.5) | 1.8 |

**Table 5b:**

| Data generated with BDP 50µg formula and a range of shaped actuator orifices (* orifice created with one drilling, all other orifices created with two drillings). | | | | | | |
|---|---|---|---|---|---|---|
| Shape | Slot | Cross | Peanut* | Peanut | Peanut | Clover Leaf |
| Area comparable to (mm): | 0.33 | 0.22 | 0.22 | 0.22 | 0.1 | 0.22 |
| Recovered (µg) | 47.49 | 50.93 | 50.70 | 51.51 | 45.12 | 47.94 |
| Delivered (µg) | 41.75 | 47.83 | 47.25 | 48.55 | 41.20 | 44.95 |
| Actuator (µg) | 5.74 | 3.13 | 3.44 | 2.97 | 3.92 | 3.01 |
| Throat (µg) | 12.44 | 11.28 | 13.32 | 5.85 | 4.92 | 6.11 |
| Stage 0 - 2 (µg) | 3.49 | 3.08 | 4.51 | 1.27 | 3.80 | 0.89 |
| Stage 0 - 2 (%) | 7.35 | 6.05 | 8.90 | 2.47 | 8.42 | 1.86 |
| FPD < 4.7µm (µg) | 25.83 | 33.45 | 29.45 | 41.42 | 32.48 | 37.94 |
| FPF<4.7 (%) | 54.39 | 65.68 | 58.09 | 80.41 | 71.99 | 79.14 |
| Dose < 1.1 µm (µg) | 14.23 | 17.03 | 18.04 | 23.37 | 18.79 | 22.11 |
| FPF<1.1µm (%) | 29.96 | 33.44 | 35.58 | 45.37 | 41.64 | 46.12 |
| MMAD (µm) | 1.3 | 1.3 | 1.3 | 1.0 | 1.2 | 1.0 |
| GSD | 2.8 | 2.4 | 3.0 | 1.9 | 2.7 | 1.8 |

**Table 6:**

| Data generated with BDP 250µg and 50µg formula and the multiple orifice actuator inserts. | | | | |
|---|---|---|---|---|
| BDP/Dose | 250µg | | 50µg | |
| Shape | 2-holes | 4-holes | 2-holes | 4-holes |
| Area comparable to (mm) | 0.12 | 0.12 | 0.12 | 0.12 |
| Recovered (µg) | 225.35 | 204.17 | 48.96 | 45.98 |
| Delivered (µg) | 207.97 | 180.95 | 46.05 | 43.65 |
| Actuator (µg) | 17.37 | 23.24 | 2.91 | 2.31 |
| Throat (µg) | 31.77 | 29.25 | 4.12 | 7.53 |
| Stage 0 - 2 (µg) | 30.42 | 24.58 | 1.97 | 4.34 |
| Stage 0 - 2 (%) | 13.50 | 12.04 | 4.02 | 9.44 |
| FPD < 4.7µm (µg) | 145.79 | 127.10 | 39.97 | 31.81 |
| FPF<4.7 (%) | 64.69 | 62.25 | 81.64 | 69.18 |
| Dose < 1.1µm (µg) | 16.11 | 17.26 | 20.64 | 16.07 |
| FPF<11µm (%) | 7.15 | 8.45 | 42.16 | 34.95 |
| MMAD (µm) | 2.7 | 2.5 | 1.2 | 1.4 |
| GSD | 1.9 | 1.9 | 2.0 | 2.7 |
| Average Shot Weight | 52.6±3.0 | 47.1±4.1 | 59.4±0.6 | 56.0±1.6 |

The shaped orifices gave very good results if the FPF_{≤4.7} is compared to the FPF_{≤4.7} obtained with a conventional actuator with equivalent orifice area. The greatest FPF_{≤4.7} for the BDP 250µg and 50µg formula was achieved with the peanut shaped orifice with an orifice area comparable to a 0.22mm conventional actuator. Very poor atomisation was observed with the peanut shaped orifice with an orifice area comparable to a 0.10mm actuator. No additional improvement in FPF_{≤4.7} is achieved with the two and four hole actuator inserts when compared to the single orifice (0.14mm diameter). The results in table 5b show that to produce the actuators with shaped orifices two laser drillings are necessary.

### Example 4

The effect of ethanol content, using the 0.22mm Bespak actuator as comparator is given in table 7a and 7b for the BDP 250µg and 50µg formulations respectively.

The effect of ethanol concentration was assessed using configuration 0.14mm actuator orifice diameter / 0.60mm orifice length (0.14/0.60) with a 50µg BDP formula containing 7%, 15%, 25% and 50% ethanol. A 250µg BDP formulation containing 15%, 25% and 50% ethanol with and without glycerol was also evaluated. The plume characteristics were assessed visually and the duration of dose generation measured acustically.

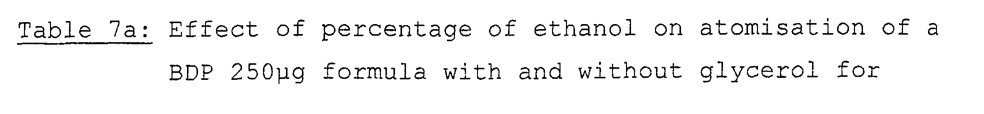

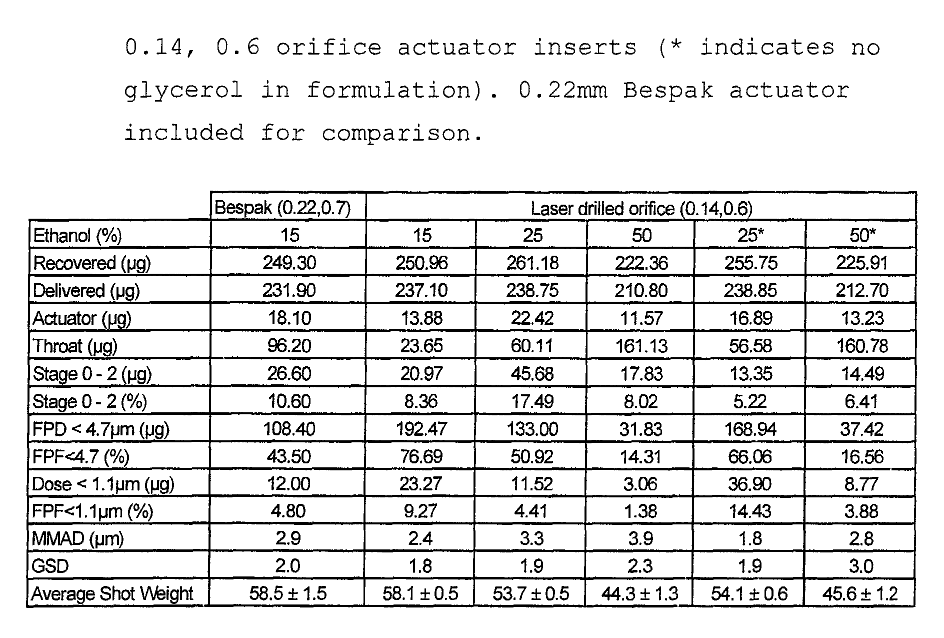

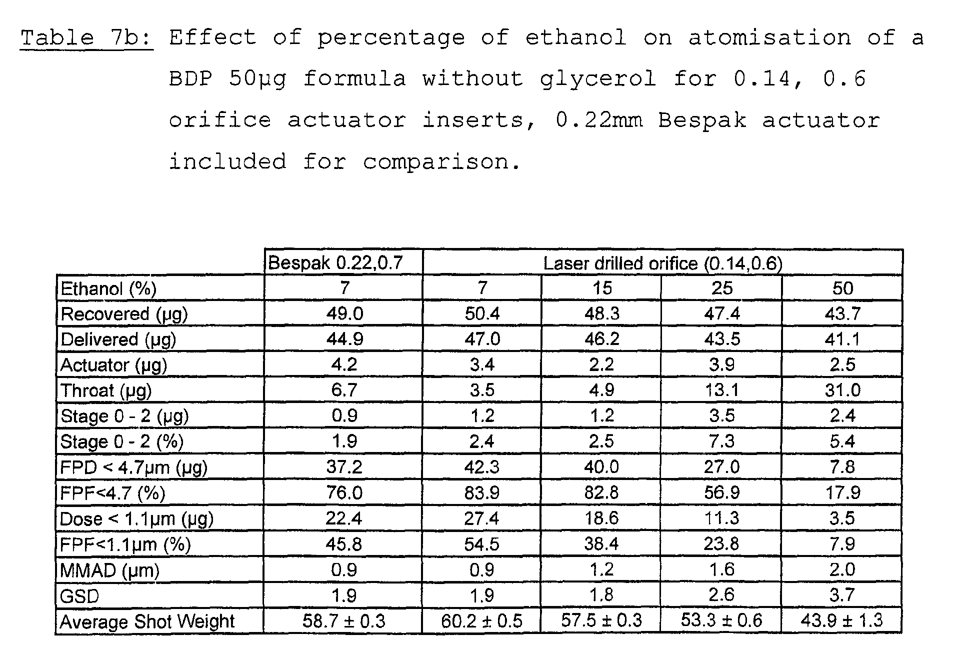

The 0.14, 0.6 orifice actuator insert was used to evaluate the effect of increasing the percentage of ethanol in the BDP 250µg formula and 50µg formula. Even with 25% ethanol in the BDP 250µg formula the 50.9% FPF_{≤4.7} obtained is greater than the FPF_{≤4.7} obtained with the 15% ethanol formula and a 0.22mm conventional Bespak actuator. However, as a consequence of increasing the ethanol content the MMAD obtained is also increased. This can be corrected by removing or altering the percentage of glycerol in the formula. This gives the formulator great scope for manipulating the formulation and achieving high drug loading and efficient atomisation if required. This is further demonstrated by the BDP 50µg results where no loss in FPF_{≤4.7} is seen when the ethanol content is increased from 7 to 15%, the MMAD however is increased from 0.9 to 1.2µm.

### Example 5

Through life testing by carrying out Andersen Cascade Impactor determinations for shots 6-15 and shots 191-200 was also carried out to evaluate actuator blockage.

The results of the through life tests with no actuator cleaning are shown in table 8.

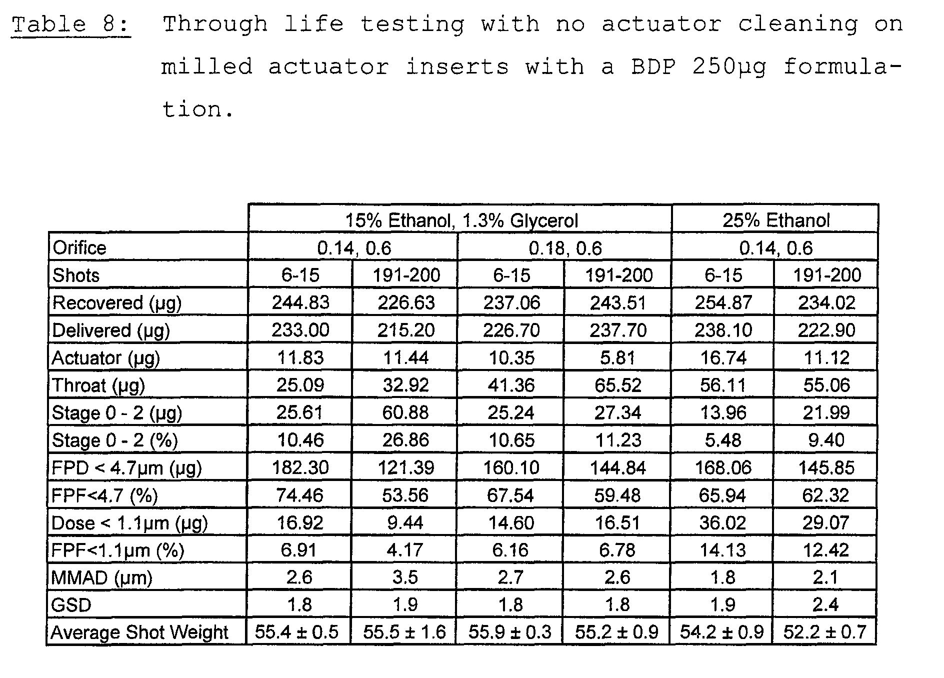

Through life testing was conducted with the 250µg BDP formula. It is clear from the results that the efficiency of atomisation is decreased at the end of the can life with the 0.14, 0.6 orifice insert. A major increase in the MMAD is also observed. The results obtained with the formula containing 25% ethanol and no glycerol shows a small increase in the MMAD and GSD. However no change in FPF_{≤4.7} is seen between beginning and end of can life. The results obtained with the 0.18, 0.6 insert show only a small decrease in efficiency through life.

### Example 6

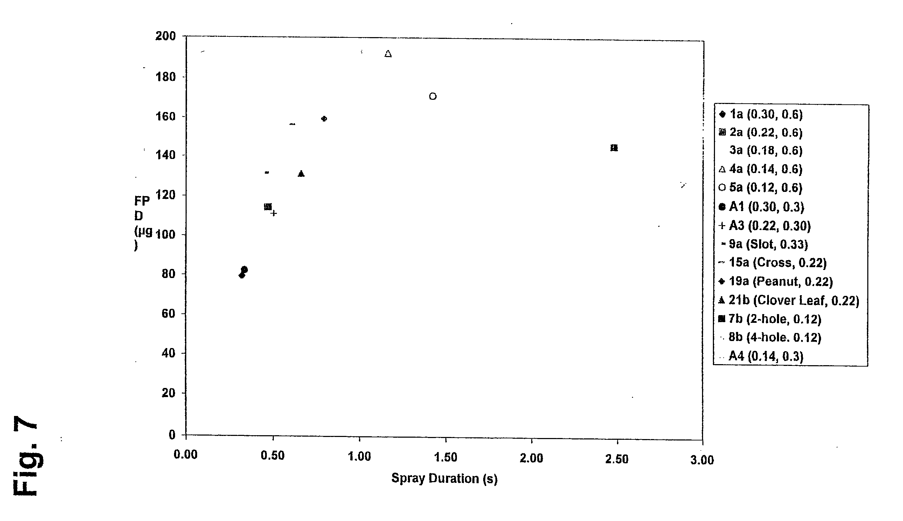

Finally, the relation of spray duration and fine particle dose for milled actuator inserts generated with BDP 250µg formulation is given in figure 7. The term "(0.14, 0.6)" describes an actuator with an actuator orifice diameter of 0.14mm and an orifice length of 0.6mm.

In summary, it can be concluded that orifice diameter decrease and length increase combine to produce fine sprays. Figure 7 clearly shows that spray duration increases as orifice diameter decreases. A spray duration of over one second can be produced with an orifice of 0.14mm diameter, with no loss in the FPF_{≤4.7} obtained.

Thus, the present invention confirms that changes in diameter and length of actuator orifices influence the speed (duration) and fine particle characteristics of clouds.

The data has revealed major new insights in the use of pMDI. Extremely efficient atomisation can be achieved with formulations containing high levels of ethanol. No loss of atomisation efficiency is seen with formulations containing up to 15% ethanol. FPF_{≤4.7} of over 50% can be achieved with formulations containing 25% ethanol. This allows the use of poorly soluble active ingredients in HFA solution formulations having a high ethanol content in order to transfer the poorly soluble active ingredient in solution. Accordingly, the present invention allows the use of new solution formulations also with poorly soluble active ingredients, which was not possible before the present invention was made.

Moreover, the data demonstrates that formulations previously unsuitable for systemic delivery (15% ethanol, 1.3% glycerol) when used with the small diameter milled inserts can produce highly efficient sprays with a much smaller MMAD, reduced throat and actuator deposition.

Preferred formulations which can be used according to the present invention are the following:

| Formulation 1: | |
|---|---|
| Salmeterol xinafoate | 3mg/can (∼ 0.025% w/v) |
| Ethanol | 20 % (w/w) |
| Glycerol | 1.3% (w/w) |
| HFA 134a | 78.7% (w/w) |

| Formulation 2: | |
|---|---|
| Salmeterol xinafoate | 3mg/can (∼ 0.025% w/v) |
| Ethanol | 15 % (w/w) |
| Glycerol | 1.3% (w/w) |
| Water | 0.5% (w/w) |
| HFA 134a | 83.2% (w/w) |

| Formulation 3: | |
|---|---|
| Fluticasone propionate | 15mg/can (∼0.12% w/v) |
| Ethanol | 30 % (w/w) |
| Glycerol | 1.3% (w/w) |
| HFA 134a | 68.7% (w/w) |

| Formulation 4: | |
|---|---|
| Fluticasone propionate | 15mg/can (∼0.12% w/v) |
| Ethanol | 25 % (w/w) |
| Glycerol | 1.3% (w/w) |
| HFA 134a | 73.7% (w/w) |

| Formulation 5: | |
|---|---|
| Fluticasone propionate | 15mg/can (∼0.12% w/v) |
| Ethanol | 20 % (w/w) |
| Glycerol | 1.3% (w/w) |
| Water | 0.5% (w/w) |
| HFA 134a | 78.2% (w/w) |

| Formulation 6: | |
|---|---|
| Mometasone popionate | 12mg/can (∼0.10% w/v) |
| Ethanol | 20 % (w/w) |
| Glycerol | 1.3% (w/w) |
| HFA 134a | 78.7% (w/w) |

| Formulation 7: | |
|---|---|
| Mometasone propionate | 6mg/can (∼0.05% w/v) |
| Ethanol | 15 % (w/w) |
| Glycerol | 1.3% (w/w) |
| Water | 0.5% |
| HFA 134a | 83.2% (w/w) |

The formulation is actuated by a metering valve capable of delivering a volume of between 50 µl and 100 µl.

The choice of the metering valve and type will be made according the knowledge of the person skilled in the art.

## Claims

1. A pressurized metered dose inhaler actuator (2) comprising a nozzle block (14; 30, 33) which comprises an actuator orifice (20) leading to an expansion chamber **characterized in that** the actuator orifice is a laser drilled orifice.

2. The actuator according to claim 1, wherein the actuator orifice diameter is in a range of from 0.10mm to 0.30mm.

3. The actuator according to anyone of claims 1 or 2, wherein the actuator orifice diameter is in a range of from 0.11mm to 0.22mm, preferably 0.11mm to 0.14mm and in particular it is 0.14mm.

4. The actuator according to claim 1 to 3, wherein the actuator orifice length is in a range of from 0.30mm to 1.7mm.

5. The actuator according to anyone of claims 1 to 4, wherein the actuator orifice length is in a range of from 0.30 to 0.60mm.

6. The actuator according to anyone of claims 1 to 5, wherein the actuator orifice is in the shape of a slot, cross, clover leaf or peanut.

7. The actuator according to anyone of claims 1 to 6, wherein the nozzle block comprises two or a plurality of orifices.

8. The actuator according to anyone of claims 1 to 7, wherein the nozzle block and/or the actuator insert piece is made of aluminium.

9. A medicinal aerosol solution formulation product comprising
a pressurized metered dose inhaler,
comprising a canister (1) equipped with a metering valve (3) and containing a medicinal aerosol solution formulation containing an active ingredient, a hydrofluorocarbon propellant, 15% or more ethanol as a co-solvent and optionally a low volatility component, and an actuator (2) as defined in anyone of claims 1 to 8.

10. The product according to claim 9, wherein the medicinal aerosol solution formulation contains at least 20% ethanol as a co-solvent.

11. The product according to claim 9 or 10, wherein the active ingredient is selected from the group consisting of beclometasone dipropionate, salmeterol xinafoate, fluticasone propionate and mometasone propionate.

12. The product according to anyone of claims 9 to 11, wherein the low volatility component is selected from the group consisting of glycerol, propylene glycol, polyethylene glycol and isopropylmyristate.

13. The product according to anyone of claims 9 to 12, wherein the propellant is selected from HFA227, HFA134a and their mixtures.

14. The product according to anyone of claims 8 to 13, wherein the medicinal aerosol solution formulation contains beclometasone dipropionate, HFA134a, 15% ethanol and 1.3% glycerol, wherein the actuator orifice diameter is 0.14mm and wherein 250µg beclometasone dipropionate are delivered per actuation.

15. A method for manufacturing a pressurized metered dose inhaler actuator comprising a nozzle block which comprises an actuator orifice leading to an expansion chamber,
**characterized in that** the method comprises a step of laser drilling said actuator orifice.

16. The method according to claim 15 **characterized in that** the method is designed so as to manufacture a pressurized metered dose inhaler actuator according to anyone of claims 1 to 8.
